# EUROPEAN PATENT SPECIFICATION

(11) **EP 4 616 861 B1**
(45) Date of publication and mention of the grant of the patent: **09.12.2026**
(21) Application number: 24209006.6
(22) Date of filing: 25.10.2024
(51) Int. Cl.: A61K 31/4706, A61P 35/00, A61P 35/04

(54) **COPPER SELECTIVE 8-AMINOQUINOLINE BASED TETRADENTATE CHELATORS FOR USE IN THE TREATMENT OF CANCER**
KUPFERSELEKTIVE TETRADENTATCHELATOREN BASIEREND AUF 8-AMINOCHINOLINE ZUR VERWENDUNG BEI DER BEHANDLUNG VON KREBS
CHÉLATEURS TÉTRADENTATES SÉLECTIFS DU CUIVRE BASÉS SUR LA 8-AMINOQUINOLÉINE POUR LEUR UTILISATION DANS LE TRAITEMENT DU CANCER

(30) Priority: 15.03.2024 WO PCT/CN2024/081837
(43) Date of publication of application: 17.09.2025
(73) Proprietor: Guangdong University of Technology, Guangzhou, Guangdong 510006 (CN); The French National Centre for Scientific Research (Centre National de la Recherche Scientifique), 75794 Paris (FR)
(72) Inventor: LIU, Yan, Guangzhou (CN); GUAN, Yingzhen, Guangzhou (CN); ANNE, Robert, Paris (FR); BERNARD, Meunier, Paris (FR)
(74) Representative: Murgitroyd & Company

(56) References cited:
- WO-A1-2017/202360
- VIRAPONG PRACHAYASITTIKUL ET AL: "8-Hydroxyquinolines: a review of their metal chelating properties and medicinal applications", DRUG DESIGN, DEVELOPMENT AND THERAPY, 1 October 2013 (2013-10-01), pages 1157, XP055642782, DOI: 10.2147/DDDT.S49763
- ZHAO JIE ET AL: "TDMQ20, a Specific Copper Chelator, Reduces Memory Impairments in Alzheimer's Disease Mouse Models", vol. 12, no. 1, 6 January 2021 (2021-01-06), US, pages 140 - 149, XP093246050, ISSN: 1948-7193, Retrieved from the Internet <URL:https://pubs.acs.org/doi/pdf/10.1021/acschemneuro.0c00621> DOI: 10.1021/acschemneuro.0c00621
- WEIXIN ZHANG ET AL: "Preparation of Tetradentate Copper Chelators as Potential Anti-Alzheimer Agents", CHEMMEDCHEM COMMUNICATIONS, WILEY-VCH, DE, vol. 13, no. 7, 23 February 2018 (2018-02-23), pages 684 - 704, XP072419414, ISSN: 1860-7179, DOI: 10.1002/CMDC.201700734

## Description

### FIELD of APPLICATION

The present invention is related to the field of anticancer drugs. More specifically, the present invention supports the use of copper chelators of the tetradentate monoquinoline (TDMQ) series as anticancer agents.

### BACKGROUND OF THE INVENTION

Cancer is the second cause of deaths after cardiovascular diseases worldwide. Over the years, many different methods of cancer treatments have been developed, including surgery, radiation therapy, chemotherapy, gene therapy and immunotherapy. Because of the complexity of cancer and the capacity of cancer cells to create resistant strains, it is absolutely necessary to create new tools in all these different therapeutic areas, including chemotherapy (V. Schirrmacher, Intern. J. Oncology, 2019, 54, 407-419; N. Vasan et al., Nature, 2019, 575, 299-309; K. Bukowski et al., Intern. J. Mol. Sci., 2020, 21, 3233).

Among all the different approaches that have been developed in chemotherapy, the category of metal ligands has not been extensively explored, despite the fact that it has been known for a long time that the content of metal ions can be different in cancer cells compared to normal ones. For example, the concentration of copper ions in metastatic carcinoma and in malignant glioma is 1.5 and 1.3 times higher than that of controls, respectively (D. Yoshida et al., J. Neurooncol., 1993, 16, 109-115). In addition, it has been evidenced that angiogenesis is a copper-dependent process (E. Urso et al., J. Vasc. Res., 2015, 52, 172-196; L. Chen et al., Signal Transduct. Target. Ther., 2022, 7, 378). Copper is involved in the tumor growth and dissemination (metastasis) of a large variety of cancer cells (S. Ishida et al., PNAS, 2013, 110, 19507-19512; G. Fnu et al., Front. Oncol. 2021, 11, 765329), and depletion of copper has been shown to inhibit angiogenesis in a wide variety of cancer cell and xenograft systems (L. Finney et al., Clin. Exp. Pharmacol. Physiol. 2009, 36, 88-94). Assays in xenograft mice (Q. Pan et al., Cancer Res., 2002, 62, 4854-4859), and some anticancer clinical trials have been performed with copper chelators, such as tetrathiomolybdate, D-penicillamine or trientine, but without real success, mainly due to the lack of metal specificity of these ligands and their potential toxicity (L. Finney et al., Clin. Exp. Pharmacol. Physiol., 2009, 36, 88-94; C. Santini et al., Chem. Rev., 2014, 114, 815-862; J. Yoshii et al., Int. J. Cancer, 2001, 94, 768-773; S. Brem et al., Neurooncol. July 2005, 246-253).

Consequently, our working hypothesis was to use highly effective and selective copper chelators as anticancer agents.

A first prior art is disclosed in an article, titled 8-Hydroxyquinolines: A review of their metal chelating properties and medicinal applications, authored by Veda Prachayasittikul,et.al., published in Drug Design, Development and Therapy, 2013, Volume 7, Pages 1157-1178**.** This document discloses 8-hydroxyquinolines, such as e.g. clioquinol, as copper chelators useful in the treatment of cancer, including breast, prostate, colon, lung, and brain cancer, for preventing cancer growth and metastasis, exerting their anticancer activity through selective antiangiogenesis activity.

A second relevant prior art is a research article, titled TDMQ20, a Specific Copper Chelator, Reduces Memory Impairments in Alzheimer' s Disease Mouse Models, authored by Jie Zhao, et al., published in ACS Chemical Neuroscience, 2021, Volume 12, Pages 140-149**.** This document discloses a new series of specific chelators of Cu(II) named TDMQ, and in particular, the specific compounds TDMQ20 , TDMQ22, for use in treating Alzheimer's disease. The document further discloses that whereas the 8-hydroxyquinolines clioquinopl and PBT2 failed in clinical trials, probably due to their lack of specificity for copper chelation, these TDMQ chelators are based on an 8-aminoquinoline skeleton with a chelating side chain at C2, which have the required structure to discriminate between copper and zinc.

A third prior art is a research article titled Preparation of Tetradentate Copper Chelators as Potential Anti-Alzheimer Agents, authored by Weixin Zhang, et al., published in ChemMedChem, 2018, Volume 13, Pages 684-704**.** This document discloses series of 8-amino-TDMQ ligands as selective copper chelators. The influence of variation of substituents or side chain length on Cu/Zn selectivity is evaluated and found superior to that of 8-hydroxyquinolines.

Also there is a patent with international publication number WO2017/202360A1 discloses tetradentate chelate monoquinoline compounds of formula (I) , and in particular the specific compounds TDMQ-5, TDMQ-9, TDMQ-10, TDMQ-12, TDMQ-13, TDMQ-16, TDMQ-19, TDMQ-20, TDMQ-22 disclosed in the examples of D4, for use in treating neurodegenerative diseases.

### Description of the related art

We initially developed a series of copper specific tetradentate ligands named TDMQ, as potential agents for the treatment of Alzheimer's disease (AD), since it has been evidenced that the homeostasis of copper is disturbed in the brain of patients with AD (see Y. Liu et al. Acc. Chem. Res., 2019, 52, 2026-2035). Then, we decided to explore the possibility of using these ligands to reduce the access of cancer cells to copper ions, since these metal ions are involved in tumor growth and angiogenesis of tumors (see Ishida *et al.* and Fnu *et al.,* cited in the section above). The TDMQ chelator series has been patented by our group (see Y. Liu et al., China (GDUT-CNRS), 27 May 2016, n° 201610369550.X, Patent number US 10,807,957 B2. Approved on October 20, 2020. Canada, patent number 3 025 406, approved on June 1st, 2021. Japan, patent number 6889825, approved on May 26th, 2021. Europe, patent application pending).

The chemical syntheses of TDMQ ligands have been reported in the patents mentioned above, and have been published (W. Zhang et al., ChemMedChem, 2018, 13, 864-704). Among all various TDMQ ligands (see above structures), we have selected TDMQ20 as the best drug-candidate because of its suitable pharmacological profile evidenced in mouse AD-models (see J. Zhao et al., ACS Chem. Neurosci., 2021, 12, 140-149).

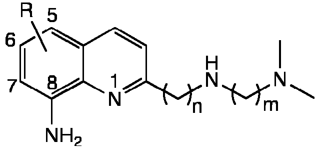

Reported General formula of TDMQ ligands.

For TDMQ20: R = 5,7-dichloro-, n = m = 2.

The cytotoxic and antiproliferative activities of TDMQ20 against several cancer cell lines are reported in the present application.

### SUMMARY OF THE INVENTION

The present disclosure provides compounds for use in methods to treat cancer or prevent metastasis.

In view of the above-mentioned medical need for more efficient compounds useful for the treatment of cancer, the present invention concerns a series of highly selective copper chelators named TDMQ having cytotoxic activities *in vitro* on several cancer cell lines.

In particular, the invention provides evidences that TDMQ20 displays various degrees of cytotoxic activity against the five tested cancer cell lines. This copper ligand was particularly efficient on the non-small cell lung carcinoma A549, the cervix cancer HeLa cells and the hepatocarcinoma HepG2, at lower concentrations than that required with the reference drug 5-FU. The cytotoxic effect of TDMQ20 against cancer cells is dose-dependent.

In addition, its selectivity for cancer cells compared to non-cancer HaCaT cells is significantly higher than that of 5-FU.

Investigations of the mechanism of action of TDMQ ligands on HeLa cancer cell lines show that TDMQ20 induces the production of ROS (reactive oxygen species), mitochondrial damage and cancer cell apoptosis. TDMQ20 also prevents cancer cell migration.

These results show that TDMQ20 has higher anti-cancer activities with a better safety window than 5-fluorouracil (5-FU), a drug which is currently used in the clinical treatment of several cancers, including breast cancer, colon or rectal cancer and stomach cancer.

### BRIEF DESCRIPTION OF THE DRAWINGS

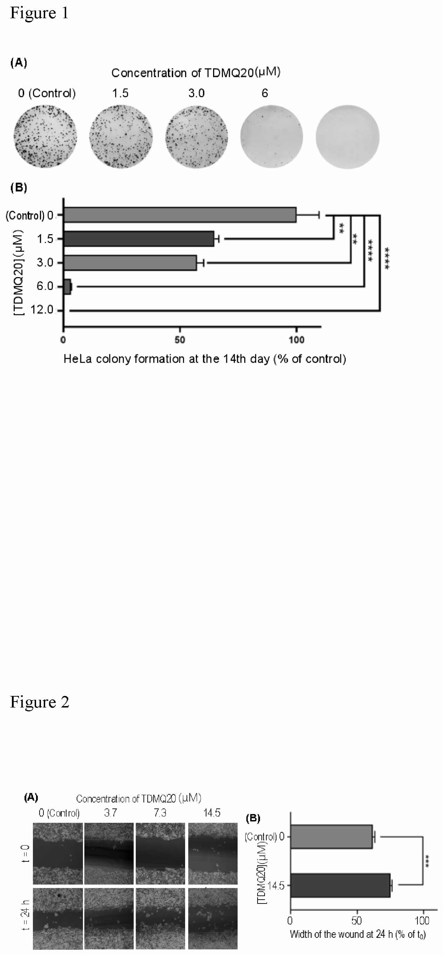

Figure 1. shows the effect of TDMQ20 on the colony-forming ability of HeLa cells at the 14^{th} day after treatment. (A) Representative images of colonies formed by HeLa cells at the 14^{th} day after treatment with increasing concentrations of TDMQ20. (B) Quantitative analysis of this clonogenicity assay. ***p <* 0.01, *****p <* 0.0001 vs. control. "Control" stands for untreated cells.
FIG. 2 shows the inhibition of TDMQ20 (14.5 µM) on the migratory ability of HeLa cells after 24h-treatment, in the wound healing assay. (A) Representative images of wounds formed by HeLa cells after treatment with TDMQ20. (B) Quantitative analysis of the wound closure upon treatment by TDMQ20. *****p* < 0.0001 vs. control. "Control" stands for untreated cells. Scale bar: 250 µm.

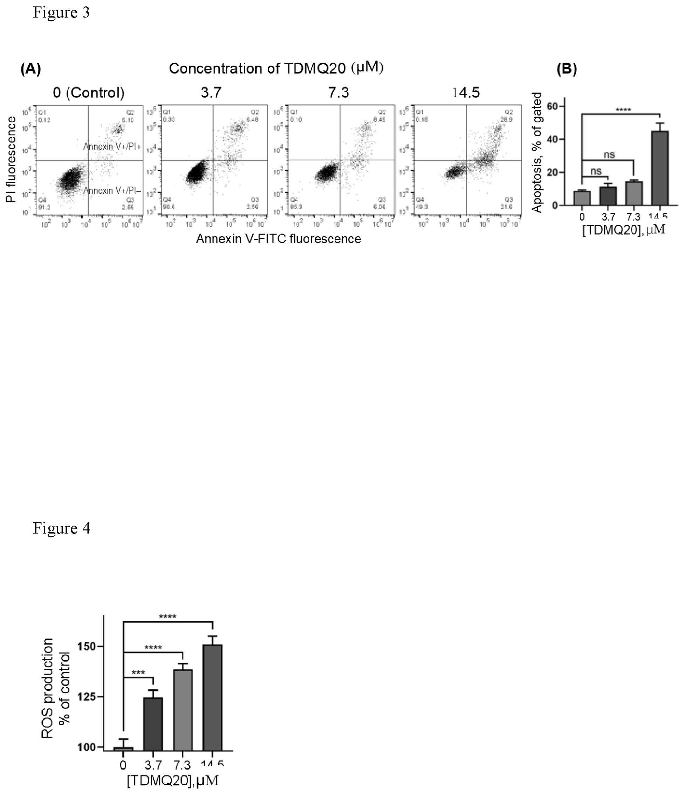

Figure 3. Pro-apoptotic activity of TDMQ20 on HeLa cells after 48 h of incubation. (A) Quantitative analysis of necrotic cells (quadrant Q1), early apoptotic cells (quadrant Q2), advanced apoptotic cells (quadrant Q3) and non-apoptotic cells (quadrant Q4), using annexin V/propidium iodide (PI) staining and detection by flow cytometry. "Control" stands for untreated cells. (B) Histograms represent the proportion of apoptotic cells (Q2 + Q3) within the flow cytometry charts; "ns" stands for *p >* 0.05, *****p* < 0.0001 *vs.* control.
Figure 4. ROS production induced by TDMQ20 in HeLa cells, detected by DCF fluorescence after 48 h of incubation. ****p* < 0.001, *****p* < 0.0001.

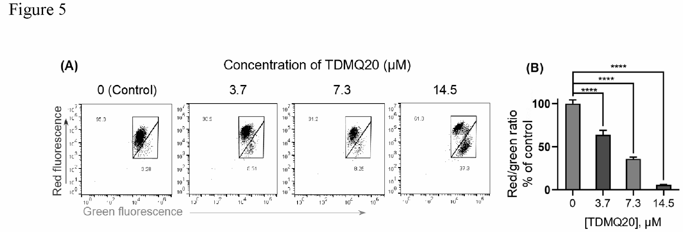

Figure 5. (A) Red/green fluorescence of HeLa cells treated with various concentrations of TDMQ20, and stained by JC-1, as an indirect measure of mitochondrial membrane potential (ΔΨm). Detection by flow cytometry. (B) Histograms presenting data of panel A as mean values of three independent experiments ± SD; *****p* < 0.0001 *vs.* control.

### DEFINITIONS

TDMQ refers to a series compound of the formula (I).

The term "copper chelators of the tetradentate monoquinoline" refers to TDMQ. Based on prior research, TDMQ as a "copper chelator" can be used for the treatment of Alzheimer's disease.

5-FU stands for 5-fluorouracil which is currently clinically used as an anticancer agent.

Cancer mainly refers to various malignant tumors. The tumor may be, but is not limited to malignant melanoma, lung cancer, breast cancer, cervix cancer, colon cancer, cutaneous melanoma, cutaneous squamous carcinoma, hepatocellular carcinoma, osteosarcoma, prostate cancer, and uveal melanoma.

The structural formula of TDMQ20 is as follows :

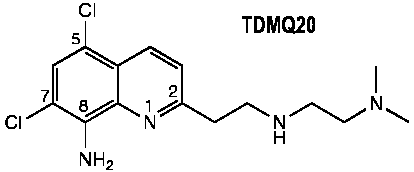

### DETAILED DESCRIPTION OF THE ILLUSTRATED EMBODIMENTS

### EMBODIMENT 1: Pharmacological activity of TDMQ ligands on different cancer cell lines

### Cytotoxicity to cancer cells, selectivity with respect to non-cancer cells

The cytotoxicity of several compounds from the TDMQ series, especially TDMQ20, was evaluated on several human cancer cell lines. The clinically used anticancer drug 5-fluorouracil (5-FU) was used as comparator.

The targeted human cancer lines were 1) the metastatic melanoma A375, 2) the non-small cell lung adenocarcinoma A549, 3) the melanoma COLO-829, 4) the cervical cancer HeLa, 5) the hepatocellular carcinoma HepG2. The human immortalized keratinocyte cell line HaCaT was used as non-cancer reference cell line.

The MTT test was used to measure the cell viability. In this test, the applied colorless tetrazolium salt is reduced by mitochondrial succinate dehydrogenase, thus producing the violet formazan derivative that is quantified by UV-visible spectrometry at 570 nm.

The cell viability was dependent on the concentration of TDMQ20, and the anti-cancer activity of TDMQ20 was quantified as the concentrations of drug inhibiting by 50 % the growth of each cell line (IC₅₀) after a 48-h incubation period.

Pharmacological results are reported in Table 1.

**Table 1. Viability of several cell lines treated with TDMQ20 for 48 h, determined by the MTT assay. Results are mean values ± SD (µM) of at least three independent experiments.**

| | IC₅₀ values (µM) ± SD at 48 h | | | | | |
|---|---|---|---|---|---|---|
| Cell lines / Drugs | A375 | A549 | COLO-829 | HeLa | HepG2 | HaCaT |
| TDMQ20 | 38.1 ± 7.0 | **16.2 ±** 2.9 | 40.2 ± 4.9 | **14.5 ±** 0.8 | **16.6 ±** 4.7 | 41.1 ± 4.8 |
| 5-FU | 68.5 ± 4.0 | 72.6 ± 10.0 | 48.7 ± 0.7 | 20.3 ± 4.6 | 46.7 ± 4.7 | 30.0 ± 2.7 |

TDMQ20 exhibited higher cytotoxic activities (lower IC₅₀ values) than 5-FU against all the tested cancer cells. Its cytotoxic activity is especially high against the non-small cell lung carcinoma A549, the cervix cancer HeLa cells and the hepatocarcinoma HepG2, with IC₅₀ values in the range 14-16 µM, lower than that of the reference drug 5-FU. The cytotoxicity of TDMQ20 on the non-cancer cell line HaCaT (IC₅₀ = 41 µM) was lower than that of the reference drug 5-FU (IC₅₀ = 30 µM). The selectivity (SI) of TDMQ20 for A549 cells, HeLa cells and HepG2 cell lines with respect to non-cancer cells HaCaT [SI = IC₅₀ (HaCaT) / IC₅₀ (cancer cell)] was ranging from 2.5 to 2.8. This safety window is better than that of 5-FU.

These results indicate the dose-dependent cytotoxic activity of TDMQ20 against several human cancer cell lines. This activity is higher than that of the reference drug 5-FU (four times higher on lung adenocarcinoma A549 cells). The cytotoxicity of TDMQ20 is selective for cancer cells with respect to non-cancer HaCaT cells (SI ≥ 2.5).

### Antiproliferative activity of TDMQ20 against HeLa cells

*Colony-forming assay.* The proliferation of HeLa cells exposed to various concentrations of TDMQ20 was evaluated at the 14^{th} day after treatment. The number of HeLa cells in cultures treated with TDMQ20 drastically decreased with increasing drug concentration from 1.5 to 12 µM. At a concentration of TDMQ20 = 12 µM, HeLa cells were not detectable.

The colony-forming ability of HeLa cells treated by TDMQ20 is reported in Figure 1.

*Wound healing assay (or "scratch assay").* The migratory ability of HeLa cells in the presence of TDMQ20 was evaluated using the wound healing assay monitored by optical microscopy. Measurement after 24 h of the width of the "wound" created in a cell monolayer quantify the migration rate of the cells to close the wound. This method mimics cell migration during wound healing *in vivo.* For cancer cells, cell migration is involved in several processes such as tumor invasion, neo-angiogenesis and metastasis (X. Wang et al., BMC Pharmacol. Toxicol. 2019, 20, 4).

*In vitro,* TDMQ20 at 14.5 µM significantly inhibited HeLa cell migration to close a wound created in a cell monolayer, indicating that the drug exhibited a cell anti-migratory effect.

The antiproliferative activity of TDMQ20 in the colony-forming assay and in the "scratch assay" suggests that TDMQ20 should be able to inhibit tumor growth and metastasis.

### EMBODIMENT 2: Mechanisms of activity of TDMQ ligands on HeLa cancer cell lines

### TDMQ20 promotes apoptosis

The pro-apoptotic activity of TDMQ20 in HeLa cells was evaluated after 48 h of incubation with the drug, using the annexin V-FITC/PI method (FITC and PI stand for fluoresceine-5-isothiocyanate and propidium iodide, respectively). After staining, live cells show little or no fluorescence (annexin V-/PI-), early apoptosis cells show green fluorescence (annexin V+/PI-), late apoptosis cells and necrosis cells show red and green fluorescence (annexin V+/PI+). Cells were detected by flow cytometry. IDEAS software was used for acquisition of data and FlowJo v. X software was used for analysis of data. Results are reported in Figure 3. The total proportion of apoptotic cells, including early apoptosis (quadrants Q2) or late apoptosis (quadrants Q3) or necrosis (quadrants Q1), accounted for 51%, 15% and 9% when HeLa cells were treated with TDMQ20 at the concentration of 14.5 µM, 7.3 µM and 3.7 µM, respectively. So, TDMQ20 induced the apoptosis of HeLa cells in a dose-dependent manner.

### TDMQ20 induces overproduction of reactive oxygen species (ROS)

Cells constantly generate reactive oxygen species (ROS) during aerobic metabolism. The ROS generation plays an important protective and functional role in the immune system. Cells are equipped with a powerful antioxidant defense system to combat excessive production of ROS. Oxidative damage, thought to play an important role in many human diseases including cancer, occurs in cells when the generation of ROS overwhelms the cell natural antioxidant defenses.

The most straightforward technique for measuring the production of reduced oxygen species in cells is the use of the cell permeable fluorescent probe 2',7'-dichlorodihydrofluorescein diacetate (H₂DCF-DA). In the presence of ROS, predominantly H₂O₂, H₂DCF is rapidly oxidized to 2',7'-dichlorofluorescein (DCF) which is highly fluorescent, with excitation and emission wavelengths of 498 and 522 nm, respectively. The stained cells were analyzed by flow cytometry and analyzed by the FlowJo v.Xsoftware (E. Eruslanov et al., Methods Mol. Biol. 2010, 594, 57-52).

Results are reported in Figure 4. The fluorescence of DCF in HeLa cells incubated with TDMQ20 at 3.7 µM, 7.3 µM and 14.5 µM, was 125%, 138% and 150% of the control cells, after 48 h of incubation with the drug. So, TDMQ20 induced a dose-dependent increase of ROS production in treated HeLa cells.

### TDMQ20 induces collapse of the mitochondrial inner transmembrane potential (ΔΨm)

The direction of the mitochondrial membrane potential (with the interior of the organelle being electronegative) allows to produce inward transport of cations and outward transport of anions, thus promoting accumulation of cations in the mitochondria. This electrochemical gradient drives the synthesis of ATP. However, during apoptosis, the mitochondrial transmembrane potential (ΔΨm) decreases since the process is associated with the opening of the mitochondrial permeability pores and loss of the electrochemical gradient. Thus, ΔΨm is an essential parameter of the mitochondrial function that can be used as an indicator of cell health since mitochondria are inherently involved in the apoptotic process of cells (D. R. Green et al., Science 1998, 281, 1309-1312.

The fluorescent membrane-permeant JC-1 dye exhibits potential-dependent accumulation in mitochondria, which forms JC-1 aggregates, and it diffuses across mitochondria to form monomeric state upon depolarization. Both JC-1 monomers and JC-1 aggregates exhibit green fluorescence (peak emission at 527 nm) due to healthy (non-apoptotic) cells and apoptotic cells, respectively. The JC-1 aggregates also exhibit red fluorescence (peak emission at 590 nm), signature of apoptotic cells. The higher is the ΔΨm, the more elevated is the red shift of the dye (more aggregates formed); vice-versa, the lower is the ΔΨm of the mitochondria and the lower is the red to green ratio of the fluorescent marker (few aggregates formed). Therefore, the ratio of red/green fluorescence intensities is significant of mitochondrial integrity and function, and mitochondrial depolarization is indicated by a reduction in the red to green fluorescence intensity ratio. (L. D. Zorova et al., Anal. Biochem. 2018, 552, 50-59 ; F. Sivandzade et al., Bio-Protocol. 2019, 9, e3128).

This method was used to evaluate the mitochondrial membrane potential in HeLa cells incubated with various concentrations of TDMQ20 for 48 h, using staining with JC-1, and quantification by flow cytometry treated with the FlowJo v. X software.

Results are reported in Figure 5. The drastic decrease of the red/green fluorescence ratio while the concentration of TDMQ20 increased (Figure 5B) indicates the collapse of the mitochondrial transmembrane potential, suggesting a drastic alteration of mitochondria.

### Comments on the results

TDMQ20 displayed various degrees of cytotoxic activity against the five tested cancer cell lines. It was particularly efficient on the non-small cell lung carcinoma A549, the cervix cancer HeLa cells and the hepatocarcinoma HepG2, with IC₅₀ values in the range 14-16 µM, significantly lower than that of the reference drug 5-FU. This effect is dose-dependent. In addition, its selectivity for cancer cells compared to non-cancer HaCaT cells is significantly higher than that of 5-FU.

Further studies carried out indicate that TDMQ20 at 3-15 µM inhibits migration of HeLa cells *in vitro,* in the colony-forming assay and in the wound-healing assay, suggesting that this drug could prevent cancer cells proliferation and metastasis *in vivo.* This effect of TDMQ20 is dose-dependent *in vitro.*

Preliminary investigations on the mechanism of action of TDMQ20 on HeLa cells *in vitro* indicate that TDMQ20 (i) enhances ROS production, (ii) drastically decreases the mitochondrial transmembrane potential ΔΨm, and (iii) induces apoptosis. In fact, the concomitance of these three phenomena is expected, because they are linked together. Mitochondrial outer membrane permeabilization and cytochrome c release promote caspase activation and execution of apoptosis in the cell. Among the first targets of activated caspases are the permeabilized mitochondria themselves, leading to the disruption of electron transport, the loss of ΔΨm, the decline in ATP levels, the production of ROS (due to electrons in the respiratory chain losing their way), and the loss of mitochondrial structural integrity. So, reduction of ΔΨm appears to be a key event during apoptosis (Q. Chen et al., Blood 1998, 92, 4545-4553; J.-E. Ricci et al., Cell 2004, 117, 773-786).

The obtained data strongly supports the selection of TDMQ20 as drug-candidate for several human cancers.

## Claims

1. A specific tetradentate copper chelator for use in treating cancer and preventing metastasis, or a pharmaceutically acceptable salt thereof, wherein the specific tetradentate copper chelator has a structure as shown in the formula (I); 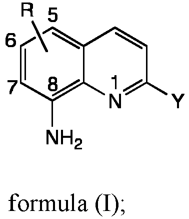 Where Y represents a group with the following formula: (CH₂)ₙ-NH-(CH₂)ₘ-N(CH₃)₂, n = m = 2, R = 5,7-dichloro-.

2. The compound for use according to claim 1, wherein the pharmaceutically acceptable salts is the hydrochloride form.

3. The compound for use according to claim 1, wherein the cancer refers to malignant tumors that may be, but is not limited to malignant melanoma, lung cancer, breast cancer, cervix cancer, colon cancer, cutaneous melanoma, cutaneous squamous carcinoma, hepatocellular carcinoma, osteosarcoma, prostate cancer, and uveal melanoma.

## Patentansprüche

1. Ein spezifischer vierzähniger Kupferchelator zur Verwendung zur Behandlung von Krebs und zur Verhinderung einer Metastasierung oder ein pharmazeutisch annehmbares Salz davon, wobei der spezifische vierzähnige Kupferchelator eine Struktur wie in Formel (I) gezeigt aufweist; 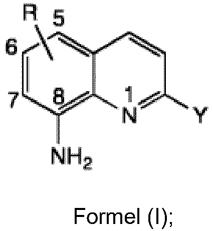 wobei Y eine Gruppe mit der folgenden Formel darstellt: (CH₂)ₙ-NH-(CH₂)ₘ-N(CH₃)₂, n = m = 2, R = 5,7-Dichlor-.

2. Verbindung zur Verwendung gemäß Anspruch 1, wobei das pharmazeutisch annehmbare Salz die Hydrochloridform ist.

3. Verbindung zur Verwendung gemäß Anspruch 1, wobei sich der Krebs auf maligne Tumoren bezieht, bei denen es sich unter anderem, ohne jedoch darauf beschränkt zu sein, um ein malignes Melanom, Lungenkrebs, Brustkrebs, Zervixkrebs, Kolonkrebs, ein kutanes Melanom, ein kutanes Plattenepithelkarzinom, ein hepatozelluläres Karzinom, ein Osteosarkom, Prostatakrebs und ein uveales Melanom handeln kann.

## Revendications

1. Un chélateur tétradenté spécifique du cuivre pour une utilisation dans le traitement du cancer et la prévention des métastases, ou un sel pharmaceutiquement acceptable de celui-ci, dans lequel le chélateur tétradenté spécifique du cuivre a une structure telle que montrée dans la formule (I) ; 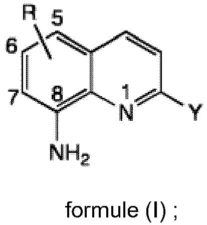 où Y représente un groupe répondant à la formule suivante : (CH₂)ₙ-NH-(CH₂)ₘ-N(CH₃)₂, n = m = 2, R = 5,7-dichloro-.

2. Le composé pour l'utilisation selon la revendication 1, dans lequel le sel pharmaceutiquement acceptable est la forme chlorhydrate.

3. Le composé pour l'utilisation selon la revendication 1, dans lequel le cancer fait référence à des tumeurs malignes qui sont notamment, sans toutefois y être limitées, un mélanome malin, un cancer du poumon, un cancer du sein, un cancer du col de l'utérus, un cancer du côlon, un mélanome cutané, un carcinome cutané squameux, un carcinome hépatocellulaire, un ostéosarcome, un cancer de la prostate, et un mélanome uvéal.
